# EUROPÄISCHE PATENTSCHRIFT

(11) **EP 0 148 385 B1**
(45) Veröffentlichungstag und Bekanntmachung des Hinweises auf die Patenterteilung: **29.10.1997**
(21) Anmeldenummer: 84114221.9
(22) Anmeldetag: 24.11.1984
(51) Int. Cl.: D06F 58/10, D06F 58/20

(54) **Trocknungsschrank und Verfahren zum Trocknen und Sterilisieren von Geweben**
Drying cupboard and method for drying and sterilizing fabrics
Armoire de séchage et procédé pour sécher et stériliser des tissus

(30) Priorität: 30.11.1983 DE 3343236; 29.11.1983 DE 3343111
(43) Veröffentlichungstag der Anmeldung: 17.07.1985
(73) Patentinhaber: Baltes, Hans, D-44267 Dortmund (DE)
(72) Erfinder: Baltes, Hans, D-44267 Dortmund (DE)
(74) Vertreter: Schulze Horn, Stefan, Dipl.-Ing. M.Sc.

(56) Entgegenhaltungen:
- DE-A- 2 110 493
- DE-A- 2 149 873
- DE-A- 2 705 116
- DE-A- 2 818 693
- DE-A- 2 919 762
- DE-B- 1 105 562
- DE-B- 1 128 601
- DE-C- 969 018
- FR-A- 1 571 470
- FR-A- 2 339 325
- FR-A- 2 367 999

## Beschreibung

Die Erfindung betrifft einen Trocknungsschrank und ein Verfahren zum Trocknen und Sterilisieren von Geweben.

Aus der DE-A-969 018 ist eine Anlage zum Desinfizieren von Gegenständen, insbesondere von Textilgut bekannt, mittels Heißluft und Dampf mit oder ohne Zusatz eines besonderen keimtötenden Mittels, unter Kreislaufführung des Desinfektionsmittels, bestehend aus einer Desinfektionskammer mit einer darin angeordneten zweiten Kammer, einer Muffel zur Aufnahme der zu desinfizierenden Gegenstände, welche mit gleichmäßig verteilt angeordneten Durchlaßöffnungen versehen ist, und einem zwischen Muffel und Kammerwand angeordneten Gebläse, dadurch gekennzeichnet, daß die nur an zwei einander gegenüberliegenden Seitenwänden mit Durchlaßöffnungen versehene Muffel innerhalb der Desinfektionskammer derart angeordnet ist, daß sie sich von der vorderen, mit einer Klapptür versehenen Kammerwandung bis zu der hinteren, ebenfalls mit einer Klapptür versehenen Kammerwandung auf dem Kammerboden anliegend erstreckt und von ihren Seitenwänden sowie ihrer Decke und den diese umgebenden Kammerwandungen ein freier, als Windkessel dienender Raum gebildet wird.

In der DE-A-969 018 ist nachteilig kein Temperaturbereich für die Sterilisierung spezifiziert, und die Trocknung findet erst nach der Sterilisierung statt. Nach der DE-A-969 018 handelt es sich um ein reines Sterilisierungsgerät und nicht um einen Trocknungsschrank.

Bekanntlich ist keiner der modernsten Waschautomaten in der Lage, die Wäsche wie früher üblich zu kochen und dadurch einen Großteil der Bakterien zu vernichten. Alle Waschmaschinen machen auch bei der Einstellung auf "Kochwäsche" vor dem eigentlichen Kochprozeß, d. h. dem Erreichen einer Temperatur von 100° C, Schluß.

Ein Großteil der Wäsche, vor allem der immer größer werdende Anteil an empfindlichem Mischgewebe, wird zum Teil nur mit 30, 40, 50 oder 60° C gewaschen, da diese empfindlichen Gewebe keine höheren Temperaturen während der mechanischen Belastung des Waschvorganges, dem Umwälzen, vertragen können.

Aus diesem Grunde können auch alle bekannten Trommeltrockner keine besonders empfindlichen Gewebe, z. B. reine Wolle oder Polyacryl, trocknen. Sie weisen weiterhin für weniger empfindliche Mischgewebe extra eine geringere Heizstufe zum Trocknen bei nur 50 bis 60 °C auf.

Aus diesem Grunde ist trotz der bis zur höchsten Reife entwickelten Waschvollautomaten und elektrischen Trommeltrockner keine Hausfrau in der Lage, ihre Wäsche biologisch und hygienisch einwandfrei sauber und rein, nämlich auch keimfrei zu machen. Bekanntlich kann man sich in Saunen, Schwimmbädern, Hotels oder bei anderen Gelegenheiten sehr leicht Fußpilz oder die verschiedensten Krankheitserreger holen, ohne daß diese in der Wäsche durch einen Waschprozeß oder modernste Trommeltrocknung vernichtet werden können.

Im Krankenhausbereich und in Arztpraxen müssen deshalb alle Ärzte- und Schwesternkittel, Handtücher und sonstige in diesem Bereich verwendete Gewebe im Anschluß an den üblichen Wasch- und Trockenprozeß auf umständliche Art und Weise zusammengefaltet in einem gesonderten Sterilisationsapparat unter bekannten Bedingungen bei keimabtötenden Temperaturen um oder oberhalb 125° C sterilisiert werden.

Die im zusammengefalteten Zustand des Gewebes der Sterilisationstemperatur ausgesetzten Kunstfasern neigen dabei zur Versprödung und beim Auseinanderlegen zu Faserbrüchen an den Knickstellen. Ein weiterer Nachteil ist der Arbeitsaufwand beim Herausnehmen der getrockneten Wäsche aus dem Trommeltrockner, Glattlegen, Zusammenfalten, Einschichten in den Sterilisator. Nach der Sterilisation ist die Wäsche wieder herauszunehmen und zu entfalten.

Ein naheliegender Verbesserungsvorschlag, wonach die Wäsche im Trommeltrockner im Anschluß an die Trocknung mit erhöhter Temperatur einer Sterilisation unterzogen wird, hat sich als undurchführbar erwiesen, weil infolge der intensiven Bewegungsabläufe und der damit verbundenen Abriebvorgänge die Fasern empfindlicher Gewebe gestaucht, gebrochen und dadurch nachhaltig geschädigt werden.

Der Erfindung liegt die Aufgabe zugrunde, unter Uberwindung der aufgezeigten Grenzen und Schwierigkeiten einen Trocknungsschrank und ein entsprechendes Verfahren anzugeben, mit welchem es gelingt, das Trocknen und Sterilisieren von empfindlichen Kunstfaser- oder Mischgeweben unter größtmöglicher Schonung der Gewebe problemlos und einfach durchzuführen. Der hierfür erforderliche Arbeitsaufwand soll dabei signifikant gesenkt werden. Eine hierfür vorgesehene Vorrichtung soll einfach und unkompliziert in der Bedienung, sparsam im Energiebedarf und mit wirtschaftlichen Mitteln herstellbar sein.

Die Lösung der Aufgabe gelingt erfindungsgemäß mit einem Trocknungsschrank zum Trocknen und Sterilisieren von Geweben, mit Einrichtungen zum Einhängen von Gewebeteilen und mit Mitteln zur Erzeugung von Warmluft zum Trocknen und Heißluft zum Sterilisieren der Gewebeteile und mit Steuermitteln, die zum Trocknen Warmluft von einer Temperatur unter 100°C und im Anschluß Heißluft zum Sterilisieren im Temperaturbereich zwischen 120°C und 145°C erzeugen.

Mit Vorteil wird hierdurch ein erheblicher Teil der bisher aufzuwendenden Arbeit für das Ausräumen des Trockners, Glattlegen, Falten und Wiedereinlegen des Gewebes in den Sterilisator erspart.

Weil sowohl der Trocknungsvorgang als auch der Sterilisierungsvorgang weitestgehend ohne daß das Gewebe einer Bewegung, Knickung oder Faltung unterworfen wird, durchgeführt werden kann, wird die größtmögliche Schonung des Gewebes erreicht, insbesondere, wenn die Sterilisierung unmittelbar im Anschluß an die Trocknung im Trockenschrank durchgeführt wird.

Es ergeben sich hierbei optimale Sterilisationsergebnisse, weil die Wäschestücke vom gewaschenen bis zum sterilisierten Zustand nicht mehr mit einer Menschenhand oder unsterilisierter Atmosphäre in Berührung kommen. Zudem wird infolge des unmittelbaren Übergangs vom Trocknen zum Sterilisieren Primärenergie gespart, sowie darüberhinaus Zeit und Arbeitskraft. Die Kittel und Tücher bleiben glatt, sie werden zudem im Gegensatz zu einer Sterilisierung im zusammengefalteten Zustand gleichmäßiger und damit wirksamer von Heißluft durchströmt und somit in der kürzest möglichen Zeit optimal sterilisiert.

Um das Gehäuse des Schrankes nicht zu heiß werden zu lassen, kann durch mindestens eine Eintrittsöffnung angesaugte, gefilterte Frischluft durch beide Außenseiten und auch durch Rückwand und Fronttür zur Abkühlung geführt werden. Dieser konstruktive Mehraufwand wird durch intensiveren Wärmetausch mehr als ausgeglichen. Die Abgabe der Abluft erfolgt dabei zweckmäßig durch wenigstens eine Öffnung im Boden oder in der Decke des Schrankes.

Weiterhin ist vorgesehen, daß zur stufenweisen Erzeugung von Warmluft zum Trocknen und Heißluft zum Sterilisieren in einem von der Luft durchströmten Heizregister dieses mit unveränderlicher Energiezufuhr bzw. unveränderter Heizleistung betrieben und mit unterschiedlichen Strömungsgeschwindigkeiten durchströmt wird. Dies wird z. B. dadurch erreicht, daß zur Sterilisierung die Ansaugung von Frischluft zur zirkulierenden Heißluft und/oder der Austritt von Abluft aus der zirkulierenden Heißluft zumindest weitgehend gedrosselt wird. Mit Vorteil wird dadurch sowohl Energie gespart als auch der Zustrom unsterilisierter Umluft verhindert.

Eine weitere Möglichkeit ist, daß das Gebläse eine Einrichtung zur Einstellung der Luftfördergeschwindigkeit, vorzugsweise in wenigstens zwei Stufen, aufweist. Durch eine Herabsetzung der Luftfördergeschwindigkeit wird eine längere Verweilzeit der Luft beim Durchströmen des Heizregisters und damit ein erhöhter Wärmeaustausch und infolgedessen eine Temperaturerhöhung der umgewälzten Luft erzielt. Weil ohnehin beim Vorgang der Sterilisation die Menge der umgewälzten Heißluft im Gegensatz zum Trocknungsvorgang stark reduziert werden kann, ist die Herabsetzung der Fördergeschwindigkeit des Gebläses bei der Erzeugung von Heißluft eine genial-einfache Maßnahme, die überdies mit einem Minimum an technischem Aufwand erreichbar ist.

Erfindungsgemäß ist der Trocknungsschrank so ausgelegt, daß die Trocknung bei Warmlufttemperaturen unter 100° C und die Sterilisierung bei Heißlufttemperaturen im Bereich zwischen 120° C und 145° C, vorzugsweise zwischen 125° C und 135° C vorgenommen wird. Somit kann bei der Sterilisierung von hängenden Gewebestücken im Trocknungsschrank die obere mögliche Grenze der Sterilisierungstemperatur zur Anwendung kommen, weil die Gewebe aufgrund der schonenden Betriebsweise außer der Temperatureinwirkung keiner weiteren schädigenden Belastung wie Bewegung, Knickung etc. unterworfen sind.

In schwierig gelagerten Fällen kann von der Maßnahme Gebrauch gemacht werden, daß der Heißluft bakterizide Stoffe in Form von Gasen, Dämpfen oder Aerosolen zugesetzt werden, z. B. halogenierte Kohlenwasserstoffe wie Tetrachlorkohlenstoff, Trichloräthylen u. ä.. Auch besteht die Möglichkeit, daß der Heißluft Chlor, vorzugsweise durch Einsprühen von chloriertem Wasser, zugesetzt wird.

Die Möglichkeit der zusätzlichen Verwendung von bakteriziden Stoffen ist dann angezeigt, wenn infolge höchster Empfindlichkeit der Gewebefasern eine Durchführung der Sterilisierung an der notwendigen Temperaturgrenze von beispielsweise 125 ° C nicht ratsam wäre. Durch Zusatz bakterizider Stoffe kann demnach eine wirksame Sterilisierung bei höchster Gewebeschonung in einem niedrigeren Temperaturniveau durchgeführt werden.

Die Einrichtungen zum Einhängen von Gewebeteilen in den Trocknungsschrank sind vorteilhaft um eine horizontale Achse schwenkbare,in einer etwa waagerechten Stellung lösbar einrastende Stangen, wobei ein die horizontale Schwenkachse bildender Gelenkzapfen ein kreisförmiges Profil mit einer eine horizontale Rastfläche bildenden Einkerbung an der der Stange abgewandten Seite aufweist, und wobei im Kopfteil der Stange eine Bohrung im Profil als Langloch mit zur Stangenachse gleichlaufender Profilachse und mit einer an der den Stangen abgewandten Seite in das Lanlochprofil hineinragenden, eine horizontale Gegenrastfläche bildenden Rastnase ausgebildet ist, wobei die Einkerbung im Zusammenwirken mit der Rastnase Rastelemente einer Sperre bilden, und daß an der den Rastelementen entgegengesetzten Seite des Langlochprofils im Kopfteil ein die Rastelemente im Eingriff haltendes federndes Rückstellelement angeordnet ist.

Durch diese Lösung ergibt sich eine denkbar einfache Ausgestaltung der verschwenkbaren Stangen für das Einhängen der Gewebestücke in den erfindungsgemäßen Trokknungsschrank. Dabei bewirkt die Ausbildung von Rastfläche und Gegenrastfläche eine vollkommen gleichmäßig waagerechte Position des nach dem Hochklappen gebildeten Stangenrostes insbesondere auch deshalb, weil das federnde Rückstellelement, z. B. ein federbelasteter Druckbolzen, die Rastelemente exakt zum Eingriff bringt. Ein weiterer Vorteil ergibt sich für die Bedienbarkeit dadurch, daß zum Entriegeln der waagerechten Stellung das Kopfteil nicht mehr angehoben werden muß, sondern ein Handdruck vom Stangenende her in Richtung des Kopfteiles genügt, um die Sperre zu entriegeln.

Eine weitere erfindungswesentliche Ausgestaltung sieht vor, daß zur Überwindung der Rückstellkraft des Rückelementes und damit zur Lösung der Sperre in der Ausnehmung des Gabelkopfes eine auf den Gelenkzapfen mit einer kreisförmigen Öffnung aufgeschobene Schaltkulisse angeordnet und mit dem Gabelkopf in einem mittig oberhalb des Langloches angeordneten Gelenk schwenkbar angelenkt und mit einem über dem Gabelkopf nach rückwärts oben überstehenden handbetätigbaren Betätigungsnocken ausgebildet ist. Dieser ist vorteilhaft mit einer napfförmigen Verbreiterung zu einer Betätigungstaste ausgebildet und mit einer Farbkennzeichnung in einer Signalfarbe, vorzugsweise mit Rot, deutlich gekennzeichnet.

Hierdurch ergibt sich eine höchst unkomplizierte und für jedermann ohne Instruktion übersichtlich erkennbare Bedienbarkeit, welche Fehlbedienung bzw. Beschädigungen praktisch ausschließt, Vorteilhaft ist insbesondere die zweifache Bedienungsmöglichkeit beim Herabklappen der Stangen, die sich dadurch ergibt, daß die Entriegelung entweder durch Druck auf das freie Ende der Stange oder durch Betätigen der Betätigungstaste mit Fingerdruck bewirkt werden kann.

Eine weitere, vorteilhafte Ausgestaltung ist, daß der Gelenkzapfen an der oberen Seite seines Profils eine gegenüber der Einkerbung um 90° C versetzte, eine vertikale Rastfläche bildende zweite Einkerbung aufweist, die im Zusammenwirken mit der Gegenrastfläche der Rastnase bei vertikal verschwenkter Lage der Stange eine Sperre gegen ein Weiterverschwenken über die Vertikale hinaus ergibt.

Dies ist eine Verbesserung gegenüber früheren Ausführungen, bei denen die herabklappende Stange bei unachtsamer Betätigung gegen die Schrankwand schlagen und dabei zumindest ein erhebliches Geräusch oder sogar Schäden verursachen konnte.

Um das Aufhängen der Wäsche auf den Stangen möglichst zu erleichtern, ist weiter vorgesehen, daß ein mit Stangen bestückter Gelenkzapfen oder eine Anordnung mehrerer Gelenkzapfen einzeln oder gemeinsam über eine oder mehrere Teleskopierschienen von einer Position innerhalb des Schrankes in eine Position außerhalb des Schrankes verschiebbar sind. Die gesamte Stangenanordnung kann damit aus dem Schrank herausgezogen und ungehindert mit Wäsche behängt werden.

Eine weitere Verbesserung dieser Anordnungen wird dadurch erreicht, daß wenigstens zwei mit Stangen bestückte Gelenkzapfen mit vertikalen Holmen mit Teleskopierschienen verbunden als vorfertigbare Montagegruppe zum Einbau in den Trocknungsschrank ausgebildet sind.

Durch eine Verbindung der untereinander liegenden Gelenkzapfen bzw. Stangenanordnungen zu einem starren Rahmen wird sowohl die Stabilität der aus dem Schrank herausziehbaren Stangenanordnung wesentlich verbessert, als auch die Fertigungs- und Montagearbeit erheblich erleichtert und damit wirtschaftlicher durchführbar gemacht.

Bevorzugt sind die Gelenkzapfen stranggepreßte Leichtmetall-Hohlprofile und die Stangen aus glasfaserverstärktem, temperaturbeständigem Kunststoff hergestellte U-Profile mit abwärts gerichteten Schenkeln und halbrundem Rücken, welche nach Art von Trägern gleicher Biegefestigkeit zum freien Ende hin verjüngt und am freien Ende mit einer Nase ausgebildet sind. Auch kann die Schaltkulisse mii Vorteil ein aus Kunststoff hergestelltes Formstück sein.

In einer besonders vorteilheifen Ausführungsform wird der beschriebene Trocknungsschrank in einem Verfahren betrieben, bei dem die Luft für die Trocknung bzw. Sterilisation teilweise im Kreislauf geführt wird, wodurch eine optimale Nutzung der eingesetzten Wärmeenergie erreicht wird.

Im folgenden wird die Erfindung in Zeichnungen in einer bevorzugten Ausführungsform gezeigt, wobei aus den Zeichnungen weitere vorteilhafte Einzelheiten der Erfindung entnehmbar sind.

Die Zeichnungen zeigen im einzelnen:
- Figur 1: einen Trocknungsschrank für die Trocknung und Sterilisation von Geweben gemäß der Erfindung,
- Figur 2: eine Ausführung des erfindungsgemäßen Trocknungsschrankes mit veränderter Luftführung,
- Figur 3: eine Einrichtung zum Aufhängen von Gewebestücken in dem Trocknungsschrank mit einem Gelenkzapfen mit einer Reihe gelenkig aufgeschobener Wäschestangen, davon vier in waagerechter, einen Aufnahmerost bildender, und eine in vertikal abgeklappter Position, in perspektivischer Ansicht,
- Figur 4: eine Gelenkanordnung im Schnitt,
- Figur 5: eine Gelenkanordnung in Draufsicht,
- Figur 6: eine Gelenkanordnung in Entriegelungsstellung, im Längsschnitt entlang der Schnittebene IV - IV in Figur 5,
- Figur 7: eine Gelenkanordnung gemäß Figur 6, ebenfalls im Schnitt der Ebene IV - IV, in abgeklapptem Zustand,
- Figur 8: einen Rahmen mit Rahmenholmen und mit einem Paar untereinader angeordneten Gelenkzapfen, aus Gründen der Übersichtlichkeit ohne Stangen dargestellt, auf Teleskopschienen aus einem Schrank herausgezogen, in perspektivischer Ansicht,
- Figur 9: einen auf Teleskopschienen gelagerten Rahmenholm, in Seitenansicht,
- Figur 10: einen Rahmenholm gemäij Figur 9 mit abgeklappten Wäschestangen, in Seitenansicht, und
- Figur 11: eine Teleskopschienenführung in annähernd natürlicher Größe, im Schnitt.

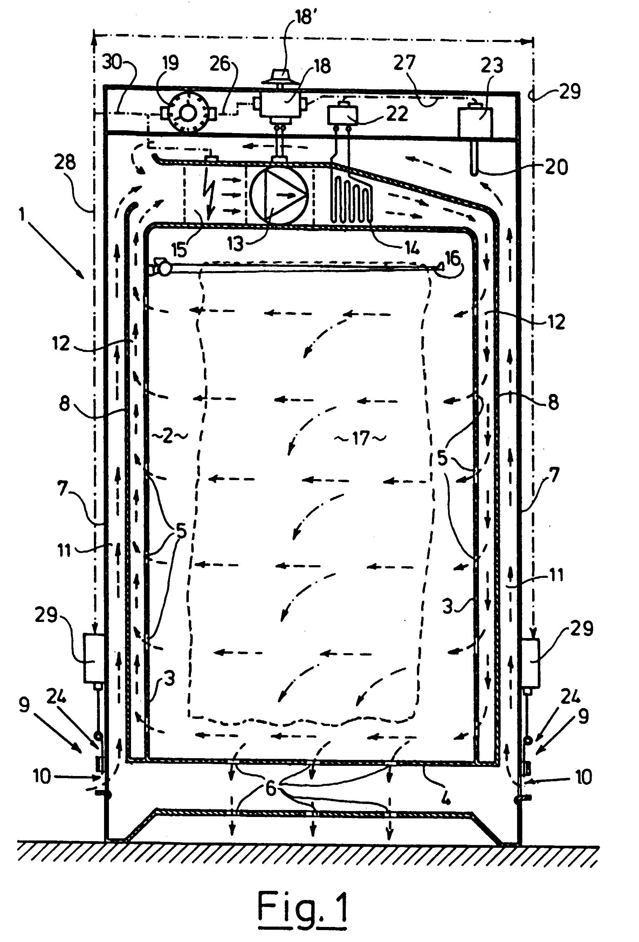

Wie Figur 1 zeigt, weist der Trocknungsschrank 1 einen Innenraum 2 auf, der Wäschestücke 17 hängend aufnimmt. Der Trocknungsschrank besitzt verschwenkbare Einhängeevorrichtungen 16 zur Behängung mit Wäsche 17. Solche Wäschehalterungen für den Trocknungsschrank sind beispielsweise durch das ältere Patent bzw. die ältere Patentanmeldung Nr. 21 49 873 bzw. P 27 05 116 bekannt geworden, worauf an dieser Stelle ausdrücklich Bezug genommen wird. Der Innenraum 2 des Trocknungsschrankes 1 ist von Wänden 3, 4 umschlossen, die ihrerseits Luftzirkulationsöffnungen 5 aufweisen. In der unteren Wand 4 sind Luftaustrittsöffnungen angeordnet. Durch die mit Pfeilen in der Figur deutlich gemachte Art der Luftführung im Querstrom und gleichmäßig in allen Ebenen des Innenraumes 2 wird die darin aufgehängte Wäsche 17 überall gleichmäßig vom Luftstrom erfaßt und durchlüftet. Der Trocknungsschrank besitzt eine äußere Verschalung 7 aus Blech oder Kunststoff. Zwischen dieser und den inneren Wänden 3 sind Wärmeaustauschwände 8 angeordnet. Im unteren Bereich 9 der äußeren Verschalung 7 befinden sich Frischlufteintrittsöffnungen 10. Durch diese strömt in Frischluftkanälen 11, welche zwischen der Verschalung 7 und jeweils einer Wärmeaustauschwand 8 ausgebildet sind, Frischluft in das System des Trocknungsschrankes 1 ein. Hierzu im Gleichstrom (linke Schrankseite) bzw. im Gegenstrom (rechte Schrankseite) strömt Systemluft aus dem Schrankinneren durch die Umluftkanäle 12. Im oberen Bereich des Trocknungsschrankes 1 befindet sich das Gebläse 13. Diesem ist das Heizregister 14 nachgeschaltet. Das Gebläse 13 ist in bevorzugter Ausgestaltung mit einer Einrichtung zur Einstellung unterschiedlicher Drehzahlen ausgestattet, beispielsweise mit einem polumschaltbaren Asynchronmotor für zwei Drehzahlbereiche. Durch den Stufenschalter 18 kann mit Hilfe des Schaltkopfes 18' manuell der gewünschte Drehzahlbereich eingestellt werden. Andererseits steht der Stufenschalter 18 über die Steuerleitung 26 mit einer Zeitschaltuhr 19 in Verbindung, mit deren Hilfe digital oder über Programmkarten ein Arbeitsprogramm gewählt werden kann, bei welchem beispielsweise eingesetzte Wäschestücke 17 nach dem Waschen in feuchtem Zustand zunächst einem Trockenprozeß bei Warmlufttemperaturen und nach Beendigung der Trocknung einem Sterilisierungsprozeß bei Sterilisierungstemperaturen unterzogen werden. Üblicherweise können Warmlufttemperaturen beim Trocknen unterhalb von 100° C gewählt sein, während zum sterilisieren eine Temperatur um 125° C erforderlich ist. Im ersteren Falle zur Erzeugung von Warmluft läuft das Gebläse 13 auf einer höheren Drehzahlstufe, wobei mehr Luft mit geringerer Temperatur umgewälzt wird. Bei Einleitung des Sterilisierungsprozesses wird das Gebläse 13 auf eine niedrigere Drehzahlstufe geschaltet, wobei weniger Luft mit höherer Temperatur gefördert wird. Um zu verhindern, daß eine obere Temperaturgrenze überschritten werden kann, ist der Trockenschrank 1 zusätzlich mit einem Überhitzungsschalter 23 ausgestattet. Dieser besitzt einen Temperatur-Sensor 20, mit dem die Temperatur einer Gasströmung an einer zweckmäßigen Stelle der Strömungskanäle, beispielsweise im Rücklaufkanal 11, ermittelt wird. Dieser Überhitzungsschalter 23 ist mit einer Steuerleitung 27 auf das Schaltrelais 22 aufgeschaltet. Dabei ergibt sich eine denkbar einfache Funktion des Überhitzungsschutzes im Go-Stop-Go-Stop-Verfahren, wobei das Heizregister 14 jedesmal bei Erreichen bzw. Überschreiten der zugelassenen oberen Temperaturgrenze abgeschaltet und nach Absinken der Temperatur auf ein zulässiges Temperaturniveau wieder zugeschaltet wird.

Beim Sterilisierungsvorgang, insbesondere für empfindliche Kunststoffaser-Gewebe wird zweckmäßig die Sterilisierungstemperatur innerhalb eines zulässigen Bereiches zwischen 125 und 130° C exakt eingehalten. Dabei empfiehlt es sich, den während der Trocknung erwünschten Zustrom von Frischluft weitgehend zu drosseln und zugleich auch die Menge der im Kreislauf geführten Systemluft stark zu reduzieren. Zu diesem Zwecke besitzt der für den Sterilisierungsprozeß ausgestattete Trocknungsschrank 1 als zusätzliche Einrichtung Absperrschieber 24 an den Lufteintrittsöffnungen 10.

Diese sind im gezeigten Beispiel mit elektromechanischen Betätigungseinrichtungen 29, vorzugsweise als Hubmagnete ausgebildet, automatisch betätigbar. Die Ansteuerung erfolgt durch das Programm des Schaltwerkes 19 über Steuerleitungen 28. Diese sind im gezeigten Beispiel rein schematisch angedeutet und im Ausführungsfall selbstverständlich im Inneren des Trocknungsschrankes üblicherweise sicher und versteckt verlegt.

Die Funktionsweise des Trocknungsschrankes 1 beim Trocknen und Sterilisieren eingehängter Wäscheteile 17, sofern sie nicht schon aus der vorgängigen Beschreibung ersichtlich ist, wird wie folgt erläutert:
Nachdem Wäscheteile 17 zum Trocknen und Sterilisieren im Trocknungsschrank 1 untergebracht sind, wird dieser geschlossen und mit Hilfe des Zeitschaltwerkes 19 ein entsprechendes Programm eingestellt, wonach zunächst der Trocknungsvorgang eingeleitet, durchgeführt und abgeschlossen, und unmittelbar anschließend der Sterilisiervorgang durchgeführt wird. Dabei erfolgt das Trocknen mit vorgegebener Trocknungszeit und Trocknungstemperatur sowie Luftmenge in bekannter Weise. Nach Abschluß der Trocknung wird durch einen entsprechenden Befehl aus dem Programm über den Stufenschalter 18 die Drehzahl des Gebläses 13 mit Hilfe des polumschaltbaren Motors reduziert. Dadurch wird weniger Luft mit höherer Temperatur durch dasmit unveränderter Energieeinspeisung betriebene Heizregister 14 gefördert. Eine Überschreitung der oberen zulässigen Temperatur wird durch den Überhitzungsschalter 23 mit dem Temperatursensor 20 verhindert. Gleichzeitig wird durch einen entsprechenden Steuerimpuls des Programmes über die Steuerleitungen 28 der Lufteinfall durch die Lufteintrittsöffnungen 10 durch die Absperrschieber 24 weitgehend gedrosselt oder verhindert.

Um die nun zirkulierende heiße Sterilisierungsluft noch mehr zu aktivieren, kann zusätzlich ein bakterizides Agens dem Heißluftstrom zugesetzt werden. Im gezeigten Ausführungsbeispiel besitzt zu diesem Zweck der Trocknungsschrank 1 eine Ozonerzeugungskammer 15, die ebenfalls vom Programm des Zeitschaltwerkes 19 über die Steuerleitung 30 angesteuert wird. Weiterhin kann der Trocknungsschrank mit einer Einrichtung zum Zugeben eines beliebigen bakteriziden Mittels versehen sein, die jedoch in der Zeichnung nicht dargestellt ist.

Die Anordnung der Ozonerzeugungskammer 15 ist selbstverständlich nicht zwingend, Weiterhin können anstelle elektromechanisch angesteuerter Absperrschieber 24 handbetätigbare Absperrschieber an den Lufteintrittsöffnungen 10, ebenso auch an den Luftaustrittsöffnungen 6 vorgesehen sein.

Der Trocknungsschrank benötigt erfindungsgemäße zur Verwendung als Sterilisierungsapparat nur einen geringfügig höheren technischen Aufwand, als in seiner bisherigen, bekannten Verwendung als reiner Trockenschrank. Infolgedessen wird die eingangs gestellte Aufgabe mit der»beschriebenen Trocknungsschrank in idealer Weise erfüllt.

Zum Aufhängen von Wäschestücken wie Kittel oder Blusen können diese auf Bügel mit verbreitertem Haken gehängt werden, so daß sich die Bügel gegenüber den Stangen nicht verdrehen können.

Die Luftführung und Anordnung der Bedienungselemente kann auch bei einem kleinen Hängeschrank so angeordnet sein, daß dieser notfalls über dem Kopf- oder Fußende einer Badewanne gehängt werden kann.

Der besondere Vorteil des Schrankes besteht weiterhin darin, daß bei ihm trotz der verwendeten hohen Temperaturen Brandgefahr ausgeschlossen ist, da sich an keiner Stelle Flusen absetzen und eine Trocknung auch ohne eine Beheizung möglich ist.

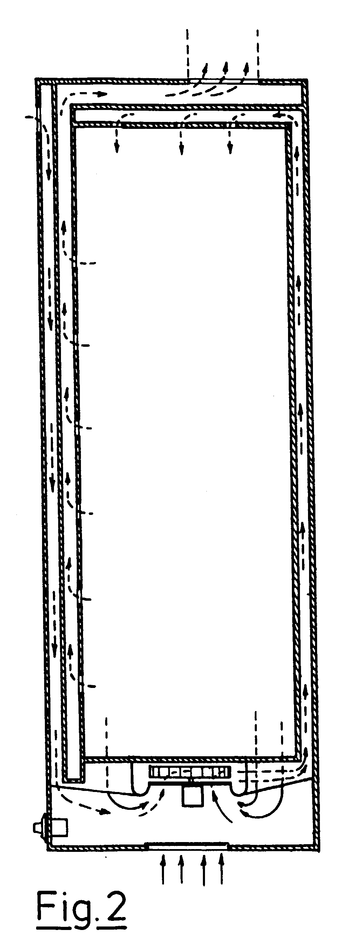

Um einen Wärmeverlust während des Trocknens und Sterilisierens und damit die Wärmebelastung der Umgebung des Schrankes so gering wie möglich zu halten, können gemäß Figur 2 nicht nur die Seitenwände, sondern auch die Rückwand, die Tür, die Decke und der Boden zweischalig ausgebildet sein und eine äußere Kaltluftführung besitzen. Auch können die Wände gqf. als Isolationswände ausgebildet sein.

Im einzelnen wird dabei die Kaltluft im Schrankboden angesaugt und strömt in der einen Schrankwand aufwärts, durchströmt dann die Decke und wird in der anderen Schrankwand abwärts geleitet und wird nach dem Durchströmen des Trockenraumes oben wieder ausgeblasen.

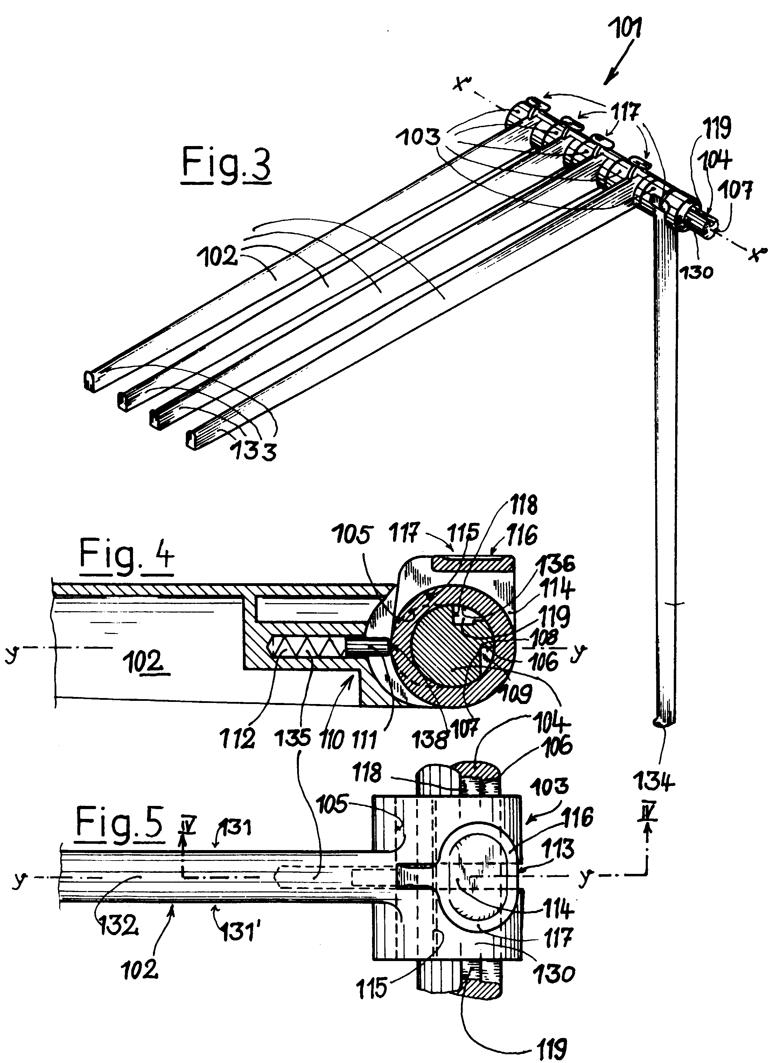

In seinem Inneren weist der erfindungsgemäße Trocknungsschrank, wie aus Figur 3 ersichtlich, eine Gelenkanordnung 101 mit Stangen 102, 102' zum Aufhängen von Wäschestücken und dergl. auf. Hiervon sind in der gezeigten Anordnung die Stangen 102 in aufnahmebereiter waagerechter Lage gezeigt, während die Stange 102' abgeklappt ist und sich in vertikaler Lage befindet. Bei der Gelenkanordnung 101 sind die Stangen 102, 102' gelenkseitig mit einem nabenförmig ausgebildeten, zur Stangenachse Y-Y querstehenden Kopfteil 103 ausgebildet. Die Kopfteile besitzen Bohrungen 130, mit denen sie um einen waagerecht verlaufenden Gelenkzapfen 104 schwenkbar angeordnet und entweder in einer von Rastelementen sperrbaren waagerechten Lage vorstehend gehalten oder nach Lösen der Sperre in die vertikale Lage abklappbar aufgenommen sind. Der Gelenkzapfen 104 besitzt ein kreisförmiges Profil mit einer eine horizontale Rastfläche 106 bildenden, parallel mit seiner Achse x-x verlaufenden Einkerbung 107 an der einer horizontal stehenden Stange 102 abgewandten Seite. Die Bohrung 130 im Kopfteil 103 ist im Profil als Langloch 105 mit zur Stangenachse y-y gleichlaufender Profilachse ausgebildet. An der stangenabgewandten Seite ragt in das Langloch 105 eine Rastnase 109 hinein, welche eine zur Stangenachse y-y parallel verlaufende Gegenrastfläche 108 bildet.

In waagerechter Lage einer Stange 102 greift die Rastnase 109 in die Einkerbung 107 ein, wobei Rastnase und Einkerbung Rastelemente einer Sperre bilden. In dieser im zusammenwirken als Sperre ineinandergreifenden Situation werden das Kopfteil 103 und der Gelenkzapfen 104 durch die Wirkung des federnden Rückstellelementes 110 gehalten. Dabei ist die Rastfläche 106 und die Gegenrastfläche 108 jeweils so ausgebildet, daß diese im Eingriff ebenflächig aneinander anliegen.

Wie aus der Darstellung der Figuren 4 und 5 weiter hervorgeht, ist das Kopfteil 103 mit einer mittigen Ausnehmung 113 als Gabelkopf ausgebildet. Im Grunde der Ausnehmung 113 ist eine Bohrung 135 in Richtung der Achse y-y verlaufend eingelassen und nimmt das Rückstellelement 110 in Form eines mit einer Sprungfeder 112 belasteten Druckbolzens 111 auf.

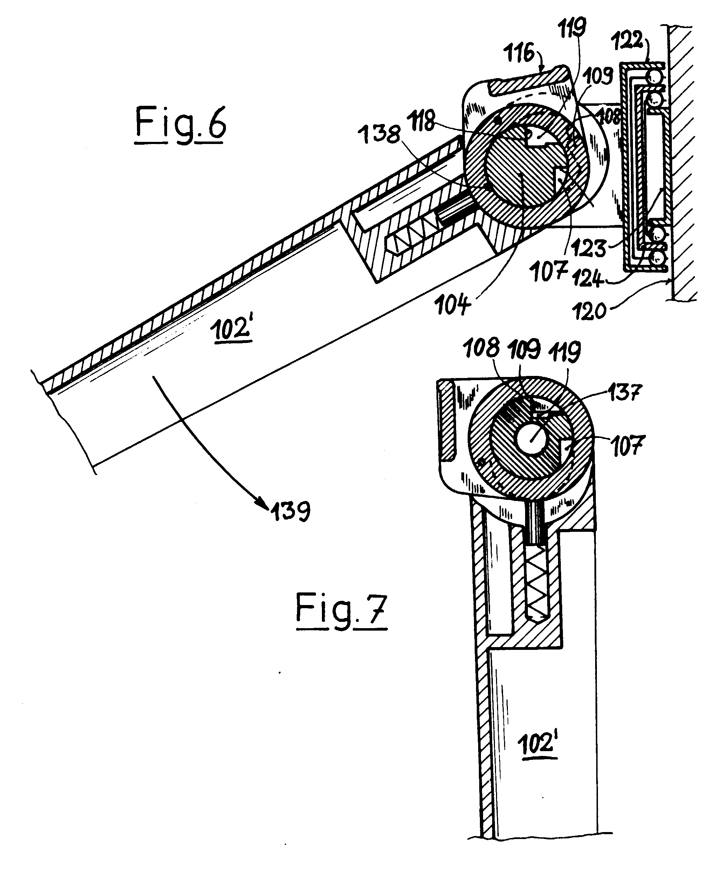

Zur Überwindung der Rückstellkraft des Rückstellelementes 110 und damit zur Lösung der Sperre ist in der Ausnehmung 113 des Gabelkopfes eine auf den Gelenkzapfen 104 mit einer kreisförmigen Öffnung 136 aufgeschobene Schaltkulisse 114 angeordnet und mit dem Gabelkopf 104 in einem mittig oberhalb des Langloches 105 angeordneten Gelenk 115 schwenkbar angelenkt. Die Schaltkulisse 114 ist nach rückwärts oben mit einem über den Gabelkopf überstehenden, handbetätigbaren Betätigungsnocken 116 ausgebildet. Dieser ist mit einer napfförmigen Verbreiterung 117 zu einer Betätigungstaste ausgebildet. Beim Niederdrücken dieser Taste mittels Fingerbetätigung macht diese gegen die Rückstellkraft des Rückstellelementes 110 eine Schwenkbewegung um das Gelenk 115 und verschiebt dadurch den Gelenkzapfen 104 relativ zur Stange 102 bzw. zum Kopfteil 103 in dem durch das Langloch 105 gegebenen Freiheitsgrad bis zum Anschlag am stangenseitigen Ende 138 des Langloches 105. Diese Position des Gelenkzapfens 104 relativ zum Langloch 105 ist aus der Darstellung in Fig. 6 deutlich erkennbar. Durch diese relative Verschiebung wird die Rastfläche 106 aus dem Eingriff mit der Gegenrastfläche 108 entriegelt und die Stange 102 kann in die vertikale Lage gemäß Pfeil 139 in Fig. 6 verschwenkt werden.

Wie aus den Figuren 4 bis 7 erkennbar, besitzt der Gelenkzapfen 104 eine zweite Einkerbung 119, welche gegenüber der ersten Einkerbung 107 um 90° versetzt angebracht ist. In diese rastet, wie aus Figur 7 erkennbar, die Rastnase 109 in der Vertikalstellung ein, wobei die Gegenrastfläche 108 gegen die vertikale Rastfläche 118 der zweiten Einkerbung 119 zur flächenhaften Anlage gebracht wird. In dieser Position findet demnach eine Verriegelung in der vertikalen Lage der Stange 102 statt, wodurch mit Sicherheit ein Überschwenken über die vertikale Lage und damit ein Anschlagen der Stangen 102 an die Innenwand des Schrankes 1 verhindert wird.

Die Einkerbungen 107 und 119 sind, wie insbesondere aus Fig. 3 bzw. 5 erkennbar, zueinander und zur Achse x-x des Gelenkzapfens 104 parallel verlaufend ausgebildet.

Zur übersichtlichen Bedienung trägt weiterhin in besonders eindrücklicher Weise bei, daß der Betätigungsnocken 116 der Schaltkulisse 114 mit einer Farbkennzeichnung in einer Signalfarbe, vorzugsweise mit Rot, ausgebildet ist. Hierdurch fällt auch dem Ungeübtesten sofort der "Druckknopf" auf, welcher zum Abklappen einer Stange 102, 102' zu betätigen ist.

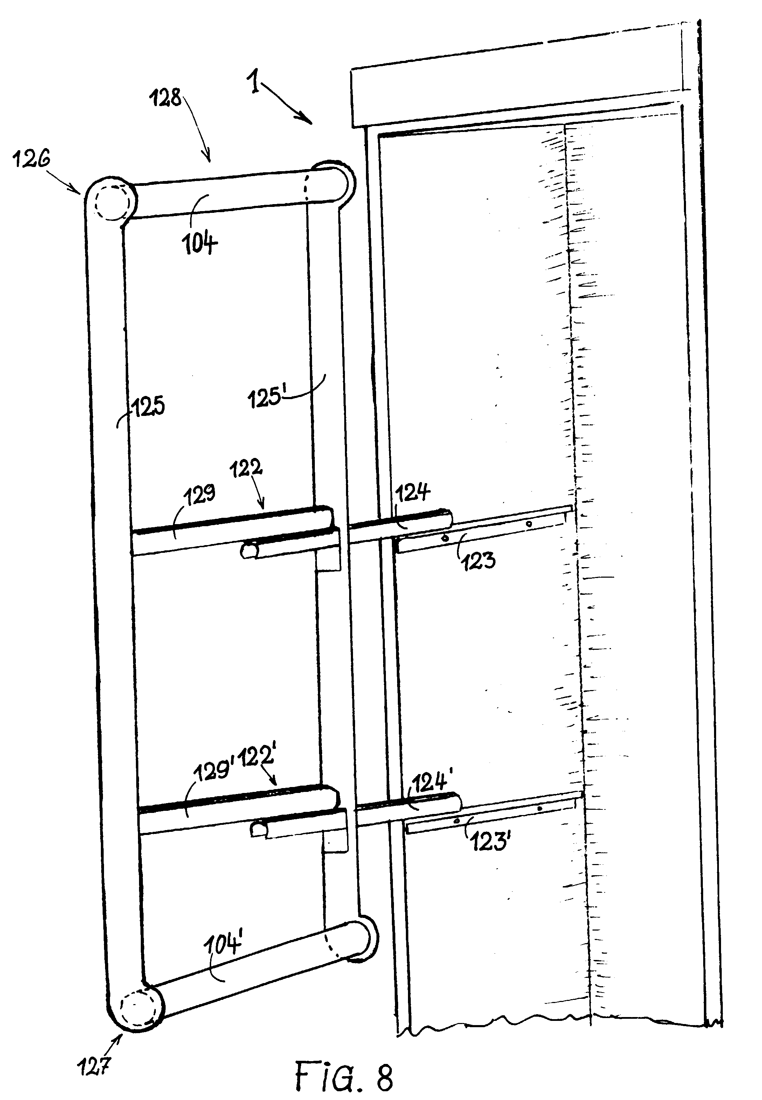

Um das Aufhängen der Wäsche auf den Stangen 102 zu erleichtern, ist der mit den Stangen 102, 102' bestückte Gelenkzapfen 104 in einer Teleskopschienenführung angeordnet. Dabei ist der Gelenkzapfen 104 mit der Teleskopschiene 122 und die Wand des Schrankes 1 mit der Teleskopschiene 123 jeweils fest verbunden. Eine dazwischen gegenüber beiden verschiebliche Teleskopschiene 124 ermöglicht eine nahezu reibungslose Verschiebung in waagerechter Richtung parallel zur x-x Achse bis zum vollständigen Herausziehen der Gelenkanordnung 101 aus dem Inneren des Trocknungsschrankes 1. Eine weitere Verbesserung gegenüber dieser Anordnung wird gemäß Darstellung in Figur 8 dadurch erzielt, daß mindestens zwei Gelenkzapfen 104, 104' mit vertikalen Holmen 125, 125' einen in sich starren rechteckigen Rahmen 128 bilden. In der Darstellung gemäß Figur 8 wurden aus Gründen der Übersichtlichkeit die Stangen 102, 102' weggelassen, welche selbstverständlich im Einbau - und Montagefall auf die Gelenkzapfen 104, 104' aufgeschoben sind. Diese sind jeweils mit den oberen Enden 126 und den unteren Enden 127 der vertikalen Holme 125, 125' fest verbunden. Eine solche Verbindung ergibt sich sehr zweckmäßig nach einem weiteren Ausgestaltungsvorschlag dadurch, daß die Gelenkzapfen 104, wie in Figur 7 dargestellt, als Profilstranggepreßte Leichtmetall - Hohlprofile hergestellt sind. Dabei kann dann durch die Profilöffnung 137 ein (nicht gezeigter) Zuganker durchgesteckt und mit den Enden 126, 127 der vertikalen Holme 125, 125' unter Vorspannung verschraubt sein. Der Rahmen 128 ist weiterhin durch Quersprossen 129, 129' versteift. Diese sind im gezeigten Beispiel als Teleskopschienen 122 ausgebildet, während im Inneren des Trocknungsschrankes 1 Teleskopschienen 123, 123' festgeschraubt sind. Die Verbindung dieser Teleskopschienen 122, 122' und 123, 123' geschieht durch Teleskopzwischenschienen 124, 124', wie dies an sich bekannt ist. Die Anordnung der Gelenkzapfen 104, 104' innerhalb des starren Rahmens 128 mit den vertikalen Holmen 125, 125' bildet als Baueinheit eine vorgefertigte Montagegruppe zum Einbau in den Trocknungsschrank 1. Damit ergibt sich eine signifikante Einsparung an Fertigungszeiten und eine unabhängige Taktfertigungsmöglichkeit für diese Baugruppe. Damit werden einerseits Fertigungskosten gespart, andererseits kann hiermit wechselnden Anforderungen an die Ausstattung eines Trocknungsschrankes mit Stangeneinbauten erheblich flexibler Rechnung getragen werden, und schlieilich erleichtert die ausziehbare Einbaugruppe dem Benutzer ganz erheblich die entsprechenden Handreichungen.

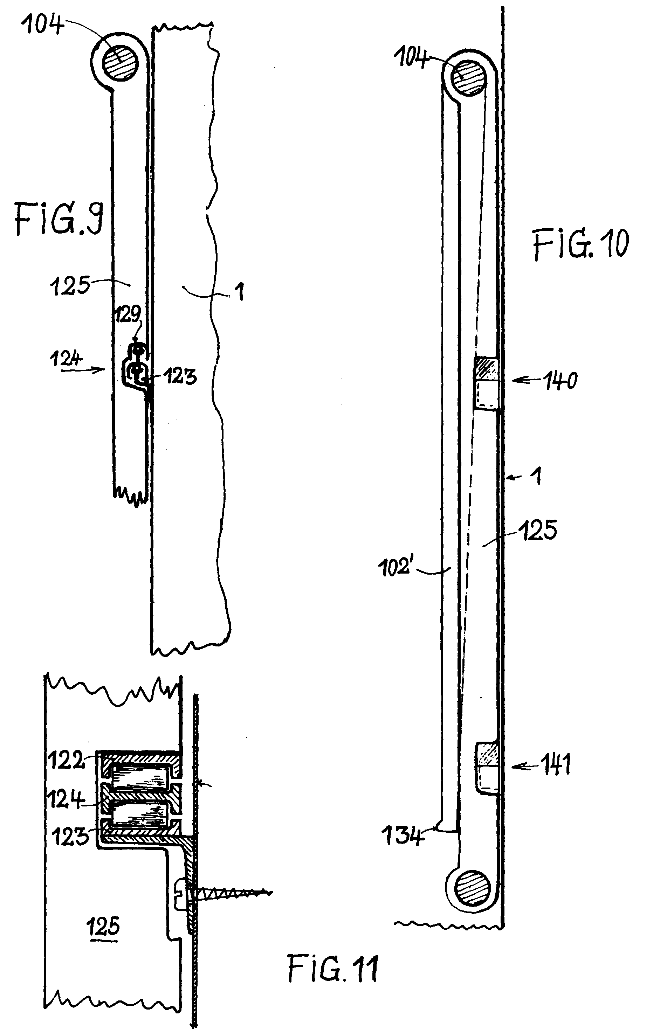

Die Anordnung eines vertikalen Holmes 125 auf Teleskopschienen 122 bis 124 zeigt Figur 9. Dabei bildet eine Sprosse 129 die oberste Teleskopschiene 122 und ein S-förmiges Winkelprofil die im Trocknungsschrank 1 befestigte untere Teleskopschiene 123 mit einer dazwischen beweglich angeordneten Teleskopzwischenschiene 124.

Figur 10 zeigt schematisch einen vertikalen Holm 125 mit in die vertikale Lage abgeklappten Stangen 102' und mit Teleskopführungen 140, 141 in Seitenansicht.

Eine der vielfältig zur Verfügung stehenden Möglichkeiten zur Ausführung einer solchen an sich bekannten Teleskopschienenanordnung zeigt Figur 11 in vergrößertem Maßstab.

Der Trocknungsschrank nach der Erfindung verbindet in ersichtlicher Weise eine optimale Funktion bei höchstem Bedienungskomfort mit einer genial - einfachen Ausführung und infolgedessen einer sehr ökonomischen Herstellbarkeit unter Überwindung der bisher bestehenden Schwierigkeiten und Grenzen.

Eine besonders große Länge der Stangen, die eine entsprechende Hängelänge ermöglicht, ergibt sich durch die Anordnung der Teleskopschienen 122, 124 und 123 übereinander. Dadurch bedingt weist der schrankseitige Vertikalholm 125' eine Aussparung zum Durchtritt der unteren Schienen 122 und 124 beim Einfahren der Stangen in den Schrankinnenraum auf.

## Patentansprüche

1. Trocknungsschrank zum Trocknen und Sterilisieren von Geweben mit Einrichtungen zum Einhängen von Gewebeteilen (17) und mit Mitteln zur Erzeugung (13, 14) und Verteilung (5, 11, 12) von Warmluft zum Trocknen,
**dadurch gekennzeichnet**,
daß er Mittel zur Erzeugung von Heißluft zum Sterilisieren der Gewebeteile (17) aufweist und daß er mit Steuermitteln (19, 26) versehen ist, die zum Trocknen Warmluft von einer Temperatur unter 100°C und im Anschluß Heißluft zum Sterilisieren im Temperaturbereich zwischen 120°C und 145°C erzeugen.

2. Trocknungsschrank nach Anspruch 1, dadurch gekennzeichnet, daß die Mittel zur Führung der Luft Verteilkanäle (11, 12) in mindestens einem Teil der Wände (3, 7, 8) sind mit wenigstens einer Frischlufteintrittsöffnung (10) und wenigstens einer Abluftaustrittsöffnung (6).

3. Trocknungsschrank nach Anspruch 1 und 2, dadurch gekennzeichnet, daß Kaltluft im unteren oder oberen Bereich der Wände (7) angesaugt und hinter diesen in Kanälen (11) zur Kühlung der Außenflächen des Trokkenschrankes (1) in den entgegengesetzten Bereich des Schrankes (1) geführt wird.

4. Trocknungsschrank nach Anspruch 1 bis 3, dadurch gekennzeichnet, daß mindestens eine der beiden Öffnungen (6, 10) ein einstellbares Drossel- und/oder Verschlußorgan (24, 29) aufweist.

5. Trocknungsschrank nach Anspruch 1 bis 4, dadurch gekennzeichnet, daß er für die Sterilisierung Heißluft im Temperaturbereich zwischen 125°C und 135°C erzeugt.

6. Trocknungsschrank nach Anspruch 1 bis 5, dadurch gekennzeichnet, daß er eine Einrichtung zum Einbringen und/oder Erzeugen eines bakteriziden Agens aufweist.

7. Trocknungsschrank nach Anspruch 1 bis 6, dadurch gekennzeichnet, daß die Einrichtungen (16) zum Einhängen von Gewebeteilen (17) in den Trocknungsschrank (1) um eine horizontale Achse schwenkbare, in einer etwa waagerechten Stellung lösbar einrastende Stangen (102, 102') sind, wobei ein die horizontale Schwenkachse bildender Gelenkzapfen (104) ein kreisförmiges Profil mit einer eine horizontale Rastfläche (106) bildenden Einkerbung (107) an der einer horizontal stehenden Stange (102) abgewandten Seite aufweist, und die Bohrung (130) im Profil als Langloch (105) mit zur Stangenachse (y-y) gleichlaufender Profilachse und mit einer an der stangenabgewandten Seite in das Langloch (105) hineinragenden, eine zur Achse (y-y) parallel verlaufende Gegenrastfläche (108) bildenden Rastnase (109) ausgebildet ist, wobei die Einkerbung (107) im Zusammenwirken mit der Rastnase (109) Rastelemente einer Sperre bilden, und daß an der den Rastelementen entgegengesetzten Seite des Langloches (105) im Kopfteil (103) ein die Rastelemente im Eingriff haltendes federndes Rückstellelement (110) angeordnet ist.

8. Trocknungsschrank nach Anspruch 7, dadurch gekennzeichnet, daß zur Überwindung der Rückstellkraft des Rückstellelementes (110) und damit zur Lösung der Sperre in der Ausnehmung (113) des Gabelkopfes eine auf den Gelenkzapfen (104) mit einer kreisförmigen Öffnung (136) aufgeschobene Schaltkulisse (114) und mit dem Gabelkopf in einem mittig oberhalb des Langloches (105) angeordneten Gelenk (115) schwenkbar angelenkt und mit einem über den Gabelkopf nach rückwärts oben überstehenden, handbetätigbaren Betätigungsnocken (116) ausgebildet ist.

9. Trocknungsschrank nach Anspruch 7 und 8, dadurch gekennzeichnet, daß der Gelenkzapfen (104) an der oberen Seite seines Profiles eine gegenüber der Einkerbung (107) um 90° versetzte, eine vertikale Rastfläche (118) bildende zweite Einkerbung (119) aufweist, die im Zusammenwirken mit der Gegenrastfläche (108) der Rastnase (109) bei vertikal verschwenkter Lage der Stange (102') eine Sperre gegen ein Weiterverschwenken über die Vertikale hinaus ergibt.

10. Trocknungsschrank nach Anspruch 7 bis 9, dadurch gekennzeichnet, daß der mit Stangen (102) bestückte Gelenkzapfen (104) oder eine Anordnung mehrerer Gelenkzapfen (104) einzeln oder gemeinsam über eine oder mehrere Teleskopschienen (122, 123) von einer Position innerhalb des Trocknungsschrankes (1) in eine Position außerhalb des Trocknungsschrankes verschiebbar sind.

11. Verfahren zum Betreiben eines Trocknungsschrankes nach den Ansprüchen 1 bis 10,
**dadurch gekennzeichnet,**
daß die Luft für die Trocknung bzw. Sterilisation teilweise im Kreislauf geführt wird.

## Claims

1. Drying cupboard for drying and sterilizing fabrics comprising means for hanging fabric parts (17), and with means for generating (13, 14) and distributing (5, 11, 12) warm air for drying,
**characterized in**
that it comprises means for generating hot air for sterilizing the fabric parts (17), and that it is provided with control means (19, 26) which for drying generate warm air with a temperature of below 100°C, and subsequently generate hot air for sterilizing in a temperature range of between 120°C and 145°C.

2. Drying cupboard according to claim 1, characterized in that the means for guiding the air are distributing passages (11, 12) in at least a part of the walls (3, 7, 8) comprising at least one fresh air inlet opening (10) and at least one exhaust air outlet opening (6).

3. Drying cupboard according to claim 1 or 2, characterized in that cold air is taken in in the lower or upper area of the walls (7), and for cooling the outer surfaces of the drying cupboard (1) is guided behind the walls in passages (11) to the opposite area of the cupboard (1).

4. Drying cupboard according to claim 1 to 3, characterized in that at least one of the two openings (6, 10) comprises an adjustable throttle and/or closing member (24, 29).

5. Drying cupboard according to claim 1 to 4, characterized in that it generates hot air in a temperature range of between 125°C and 135°C for sterilizing.

6. Drying cupboard according to claim 1 to 5, characterized in that it comprises an apparatus for introducing and/or generating a bactericidal agent.

7. Drying cupboard according to claim 1 to 6, characterized in that the means (16) for hanging fabric parts (17) in the drying cupboard (1) are bars (102, 102') which are pivotable about a horizontal axis, and will detachably snap-in in an approximately horizontal position wherein a pivot pin (104) defining the horizontal pivot axis comprises a circular profile with a notch (107) forming a horizontal snap-in surface (106) at the side facing away from a horizontally positioned bar (102), and the bore (130) is designed in the profile thereof as an elongated hole (105) with a profile axis coincident with the bar axis (y-y), and with a snap-in lug (109) protruding into the elongated hole (105) at the side facing away from the bar, and forming a counter snap-in surface (108) extending in parallel with the axis (y-y), wherein the notch (107) in cooperation with the snap-in lug (109) forms snap-in elements of a lock, and that at the side of the elongated hole (105) facing away from the snap-in elements in the head part (103) a resilient resetting element (110) is positioned keeping the snap-in elements engaged.

8. Drying cupboard according to claim 7, characterized in that for overcoming the resetting force of the resetting element (110) and thereby for releasing the lock in the recess (113) of the bifurcate head a shifting gate (114) pushed onto the pivot pin (104) with a circular opening (136) is provided, and is pivotably connected with the bifurcate head in a joint (115) positioned centrally above the elongated hole (105), and is provided with a manually operable actuating cam (116) on the top thereof protruding backwards.

9. Drying cupboard according to claim 7 and 8, characterized in that the pivot pin (104) at the upper side of the the profile thereof comprises a second notch (119) offset by 90° in relation to the notch (107) and forming a vertical snap-in surface (118) with the second notch in cooperation with the counter snap-in surface (108) of the snap-in lug (109) providing a lock against further pivoting the bar (102') beyond the vertical position thereof.

10. Drying cupboard according to claim 7 to 9, characterized in that the pivot pin (104) provided with bars (102), or an arrangement of several pivot pins (104) piece by piece or in total by means of one or several telescoping rails (122, 123) are displaceable from a position within the drying cupboard (1) to a position outside the drying cupboard.

11. Method for operating a drying cupboard according to the claims 1 to 10, characterized in that the air for drying or sterilizing, respectively, is partly guided in circuit.

## Revendications

1. Armoire de séchage pour sécher et stériliser des tissus, munie d'organes pour suspendre des pièces de tissu (17) et de moyens pour produire (13, 14) et pour répartir (5, 11, 12) de l'air chaud de séchage,
caractérisée en ce qu'elle comporte des moyens pour produire de l'air très chaud destiné à stériliser les pièces de tissu (17), et en ce qu'elle est munie de moyens de commande (19, 26) qui produisent, pour le séchage, de l'air chaud à une température inférieure à 100°C et en dérivation de l'air très chaud pour stériliser dans une gamme de température comprise entre 120°C et 145°C.

2. Armoire de séchage selon la revendication 1, caractérisée en ce que les moyens pour guider l'air sont constitués par des canaux de répartition (11, 12) dans au moins une partie des parois (3, 7, 8) avec au moins une ouverture (10) d'entrée d'air frais et au moins une ouverture (6) de sortie d'air usé.

3. Armoire de séchage selon la revendication 1 et 2, caractérisée en ce que l'air froid est aspiré dans la zone inférieure ou supérieure des parois (7) et est refoulé derrière celles-ci dans des canaux (11) pour refroidir les surfaces extérieures de l'armoire de séchage (1), dans la zone opposée de l'armoire (1).

4. Armoire de séchage selon la revendication 1 à 3, caractérisée en ce qu'au moins l'une des deux ouvertures (6, 10) comporte un organe réglable d'étranglement et/ou d'obturation (24, 29).

5. Armoire de séchage selon la revendication 1 à 4, caractérisée en ce qu'elle produit, pour la stérilisation, de l'air très chaud dans une gamme de températures comprise entre 125°C et 135°C.

6. Armoire de séchage selon la revendication 1 à 5, caractérisée en ce qu'elle comporte un organe pour apporter et/ou pour produire un agent bactéricide.

7. Armoire de séchage selon la revendication 1 à 6, caractérisée en ce que les organes (16) pour suspendre les pièces de tissu (17) dans l'armoire de séchage (1), sont des barres (102, 102') susceptibles de pivoter autour d'un axe horizontal, et de se verrouiller de façon amovible dans une position sensiblement horizontale, en ce qu'une broche d'articulation (104) formant l'axe d'articulation horizontale, présente un profil circulaire avec une entaille (107), formant une surface de verrouillage horizontale (106) sur l'une des faces situées à l'opposé de la barre placée horizontalement, et en ce que l'alésage (130) présente en profil la configuration d'un trou oblong (105) avec un axe de profil confondu avec l'axe (y-y) des barres et avec, du côté situé à l'opposé de la barre, un bec de verrouillage (109), faisant saillie dans le trou oblong (105), et formant une contre-surface de verrouillage (108) courant parallèlement à l'axe (y-y), en ce que l'entaille (107) forme, en coopération avec le bec de verrouillage (109), des éléments de verrouillage d'un verrou, et en ce que, sur la face du trou oblong (105) opposée aux éléments de verrouillage, dans la partie de tête (103), est disposé un élément élastique de rappel (110) maintenant en contact les éléments de verrouillage.

8. Armoire de séchage selon la revendication 7, caractérisée en ce que, pour surmonter la force de rappel de l'élément de rappel (110), et pour déverrouiller ainsi le verrou dans la cavité (113) de la tête de fourche, une coulisse de commutation (114) glissée sur la broche d'articulation (104) par une ouverture circulaire (136) est articulée pivotante par une articulation (115) disposée au milieu, au-dessus du trou oblong (105), et est constituée par une came d'actionnement (116) susceptible d'être actionnée manuellement et faisant saillie vers l'arrière au-dessus de la tête des fourche.

9. Armoire de séchage selon la revendication 7 et 8 caractérisée en ce que la broche d'articulation (104) comporte, à la face supérieure de son profil, une deuxième entaille (119) décalée de 90° par rapport à l'entaille (107) et formant une surface de verrouillage vertical (118), cette deuxième entaille assurant, en coopération avec la contre-surface de verrouillage (108) du bec de verrouillage (109), pour la position verticale basculée de la barre (102'), un verrouillage à l'encontre d'une poursuite du basculement au-delà la verticale, vers l'extérieur.

10. Armoire de séchage selon la revendication 7 à 8, caractérisée en ce que la broche d'articulation (107), équipée des barres (102), ou bien un agencement de plusieurs broches d'articulation (104) est(sont) susceptible(s) de coulisser seule(s) ou en commun sur un ou plusieurs rails télescopiques (122, 123), depuis une position à l'intérieur de l'armoire de séchage, jusqu'à une position à l'extérieur de l'armoire de séchage.

11. Procédé d'exploitation d'une armoire de séchage selon les revendications 1 à 10, caractérisé en ce que l'air pour le séchage ou pour la stérilisation est guidé partiellement dans le circuit.
